Europäisches Patentamt

European Patent Office

Office européen des brevets

(19)

(11) Publication number: **0 462 534 A2**

# EUROPEAN PATENT APPLICATION

(21) Application number: **91109883.8**

(22) Date of filing: **17.06.91**

(51) Int. Cl.⁵: **C12N 15/31**, C07K 13/00, A61K 39/10, A61K 39/116, C12N 1/21, //(C12N1/21, C12R1:01)

(30) Priority: **21.06.90 IT 2071090**

(43) Date of publication of application:
**27.12.91 Bulletin 91/52**

(84) Designated Contracting States:
**AT BE CH DE DK ES FR GB GR IT LI LU NL SE**

(71) Applicant: **SCLAVO S.p.A.**
**Via Fiorentina 1**
**I-53100 Siena(IT)**

(72) Inventor: **Marsili, Ilio**
**Via delle Province, 8**
**I-53100 Siena(IT)**
Inventor: **Ruggiero, Paolo**
**Piazza San Faustino, 17**
**I-01100 Viterbo(IT)**
Inventor: **Rappuoli, Rino**
**Via Calamandrei, Localita Quercegrossa**
**I-53035 Monteriggioni, (Siena)(IT)**

(74) Representative: **Gervasi, Gemma et al**
**NOTARBARTOLO & GERVASI Srl Viale**
**Bianca Maria 33**
**I-20122 Milan(IT)**

(54) Acellular anti-pertussis vaccines.

(57) Novel acellular anti-pertussis vaccines are described, which comprises a non-toxic double mutant of pertussis toxin (PT), filamentous haemagglutinin (FHA) and the 69 kilodaltons protein (69Kd) taken alone or in combination with the diphtheria and tetanus anatoxin. The completely non-toxic double mutant of the PT is characterized by the fact that is comprises in the amino acid sequence of the S1 subunit the substitution of Glu 129 and of one of any other amino acid of the subunit, whose substitution brings about a decrease in toxicity, by natural amino acids. Moreover there are described purification methods for, and the characterisation of the FHA and the 69 Kd protein expressed by transformed B. pertussis W28-9K/129G strains, which produce the PT double mutant and by transformed B. pertussis W28-ΔTox which do not produce the PT. These proteins are particularly suitable for preparing safer and more immunogenic acellular anti-pertussis vaccine.

EP 0 462 534 A2

Technical field of the invention.

The present invention relates to acellular anti-pertussis vaccines comprising a non-toxic double mutant of pertussis toxin filamentous haemagglutinin (FHA), 69 kilodaltons (69Kd) protein and pharmaceutical acceptable vectors, optionally in combination with diphtheria and tetanus anatoxins.

State of the art.

Pertussis (whooping cough), i.e. infective disease of bacterial origin characterized by paroxysms of convulsive coughing and severe respiratory symptomatology, affects subjects of any age and, in the early childhood, is fatal in the 0.5 % of the cases. Bordetella pertussis, which is the aetiological agent of pertussis, produces during the virulent phase (Phase I) a series of toxic components, among which the pertussis toxin (PT) represents not only the main pathogenic agent for the disease, but also the main immunogen. The PT, which is structured as an esamer consisting of five different subunits (S1, S2, S3, S4 and S5) is capable of eliciting in laboratory animals antibody levels sufficient to impart protection against pertussis. The occurrence of said infection can be effectively controlled by immunisation of a subject with a suitable vaccine. At present a cellular vaccine, namely consisting of whole virulent B. pertussis cells treated with merthiolate and killed at 56°C, is used. Said vaccine, though imparting a protective immunity, may nevertheless induce undesirable site effects ranging from simple swellings, erythema and fever to convulsions and cerebral reversible and irreversible injury. Therefore there have been proposed in the prior art acellular vaccines consisting of one or more antigenic proteins, among which the pertussis toxin detoxified with a range of chemical reagents such as formaldehyde [Sato et al., (1983) Infect. Immun. , 41, 313-320], glutaraldehyde [Quentin - Millet et al. , (1988), J. Biol. Stand., 16, 99-108], tetranitrometane [Siber et al., (1988), Winberry et al., (1988), International Workshop of Bordetella pertussis, Hamilton, MO], trinitrobenzensolphonic acid [Fisch et al., (1984) Infect. Immun., 44, 1-16] hydrogen peroxide [ Sekura et al., (1983), Ubfect. Immun., 113, 806-813]. 44,1-16 113, 806-813].
Such detoxifying methods show however the following drawbacks:
- reversion to toxicity; in fact, acellular vaccines consisting of the sole formaldehyde detoxified PT [ Sato Y. et al., Lancet i, (1984), 122-126], although capable of protecting 80% of the children against the disease and 50%-60% against the infection [Ad hoc Group for the Study of Pertussis Vaccines, Lancet i, (1988), 959-960], show nevertheless a few occurrences of reversion to toxicity [Storsaeter J. et al. , Pediatr. Infect. Dis. J. , 7, (1988), 637-645]; - - reduced immunogenicity due to the drastic condition necessary for the detoxifying phase;
- lack of repeatability of the detoxified products;
- necessity for assays, whose execution is highly time consuming, to evaluate the extent of the reversion for each preparation; and finally
- The hazard in handling large amounts of toxic material for the manufacturing personal.
Pertussis toxin toxicity is known to be mediated by the ADP-ribosyltransferase activity of the S1 subunit. In order to obtain molecules with modified toxicity vis-a-vis that of the PT wild type, thus suitable for the preparation of anti-pertussis vaccines free of the aforementioned drawbacks, mutants of the pertussis toxin have been devised and produced, as disclosed in the (Italian) patent application No.19286 of 07.02.90, which comprise one or more amino acid substitutions within the sequence of the S1 subunit of PT. An example of the decrease in toxicity of some of such mutants is reported in Table I

TABLE I

Percent correlation between the _in vitro_ enzyme activity and toxicity of some PT mutants.

_____

| Mutant | | ADP-ribosylation | toxicity on CHO |
|---|---|---|---|
| PT | | 100 | 100 |
| PT-9K | (Arg9->Lys) | 0.1 | 0.1 |
| PT-13L | (Arg13->Leu) | 2 | 25 |
| PT-26I | (Trp26->Ile) | N.D. | 10 |
| PT-129G | (Glu129->Gly) | 0.5 | 5 |
| PT-9K/129G | | <0.01 | <0.0001 |
| PT-13L/129G | | <0.01 | <0.0001 |
| PT-26I/129G | | <0.01 | <0.0001 |

Detailed description of the invention.

It has now been surprisingly discovered and represents object of the present invention that pertussis toxin mutants where, the amino acid Glu129 in the S1 subunit and one of any other amino acid whose substitution brings about a decrease in toxicity, are both substituted by a natural amino acid are practically completely non-toxic and therefore they are suitable for preparing acellular anti-pertussis vaccines.

As specific, however not limiting examples of the present invention, there are described vaccines comprising mutants wherein the following substitutions have been introduced: Arg 9 and Glu 129 or Trp 26 and Glu 129 or Arg 13 and Glu 129 or Asp 11 and Trp 26 and Glu 129. In the above cited examples said amino acids are substituted as follows: Glu 129 -> Gly, Arg 9 -> Lys, Trp 26 -> Ile, Arg 13 -> Leu, Asp 11 -> Ser.

It was moreover discovered, and it is further object of the present invention that, in order to confer a more complete protection against pertussis, further Bordetella antigens, such as the FHA and the 69 kilodaltons (69Kd) outer membrane protein from B. pertussis may optionally be taken into account in formulating acellular vaccines; more specifically the FHA and 69Kd produced by Bordetella strains deleted of the PT gene (see IT-A-19286).

Purpose of the present invention is therefore developing immunogens suitable for preparing anti-pertussis vaccines free of the disadvantages known in the prior art. This purpose is achieved according to the present invention by availing novel Bordetella strains capable of expressing an immunogenically active pertussis toxin mutant free of toxicity, the FHA and the 69Kd, and/or Bordetella strains ΔTox, which are incapable of producing the pertussis toxin but nevertheless capable of producing the FHA and the 69Kd for the manufacture of effective acellular anti -pertussis vaccines.

A further object of the present invention is the use of said antigens, optionally treated with formaldehyde, for preparing effective acellular anti- pertussis vaccines.

A still further object of the present invention are immunogenic formulations suitable as anti- pertussis vaccines capable of inducing, on animal model, a protective response effective against infections from virulent B.pertussis; said vaccines contain an immunogenically effective amount of a PT mutant alone and/or FHA and/or 69Kd or are furthermore combined with the diphtheria and tetanus anatoxins, whereby said antigens are optionally stabilized with formaldehyde. Further objects and embodiments of the present invention will be evident in the light of following description and examples which are provided for illustrative, but not limiting purpose.

The preferred antigens according to the present invention are the pertussis toxin mutant characterized in that the amino acid residues Arg 9 and Glu 129 are deleted and respectively replaced by the amino acid residues Lys and Gly (PT-9K/129G), the FHA and the 69Kd. According to the previous invention disclosed in the Italian patent application No. 19286 of 07.02.1990, Bordetella strains capable of producing the aforementioned antigens are obtained by a process comprising:

a) selecting Bordetella wild type strains resistant to at least one antibiotic;

b) replacing by homologous recombination in strains obtained in stage (a) the chromosomal gene coding for the pertussis toxin with a gene encoding a different protein;

c) selecting the Bordetella strains deleted of the PT gene ($\Delta$Tox) obtained in stage (b);

d) mutagenizing the pertussis toxin gene isolated from B. pertussis;

e) introducing said mutagenized gene into a suitably modified plasmid nonreplicable in Bordetella;

f) introducing said plasmid into Bordetella ($\Delta$Tox) strains selected in stage (c) by conjugation; and finally

g) isolating the Bordetella strains transformed by homologous recombination with the gene of the mutagenized pertussis toxin.

According to the present invention the double mutant PT-9K/129G or PT-13L/129G or PT-26I/129G and FHA are purified from the acellular culture medium while 69Kd is purified from the cellular part.

According to the present invention the chimico-physical features and the biological and immunological activity of said proteins have been tested in vitro and in vivo. With regard to the chemical-physical properties, the analysis by SDS polyacrylamide gel electrophoresis (PAGE) and the Western Blotting analysis show the absence of contaminating proteins and a profile conforme to the molecular weight of said proteins, while the amino acid analysis puts in evidence a composition in agreement with the values predictable on the basis of the known amino acid sequence. According to the present invention the immunogenic properties of the double mutant PT-9K/129G, of the FHA, and of the 69Kd are assayed in vivo as reported in the examples hereinafter. The results show that said antigens are capable of eliciting specific antibodies with high antibody titre and imparting an effective protection against virulent B. pertussis infections when used either alone or in combination with diphtheria and tetanus anatoxin. Said antigens obtained according to the present invention are therefore the best candidate for developing effective anti-pertussis vaccines. Immunogenic formulations suitable as anti-pertussis vaccines may be prepared according to the present invention by adding said antigens to a pharmaceutically acceptable carrier selected among those generally used as vehicles for immunogenic materials in humans. Example of such carriers is the saline solution. The antigenic product may be present as a solution or a suspension in the carrier. Said formulations may moreover comprise an adjuvant to stimulate the immunoresponse and thus to improve the effectiveness of the vaccine. Suitable adjuvants for the purpose of the present invention are for example aluminium phosphate (AlPO$_4$), aluminium hydroxide [Al(OH)$_3$] interleukin-1 or 2 or peptide fragments thereof. Immunogenic formulations suitable as anti-pertussis vaccines comprise, generally, an antigen end concentration so selected to confer an effective immunity against pertussis. After formulation the vaccine may be put into a sterile vessel and stored at different temperatures or lyophilized.

In order to elicit a protective immunity against pertussis one or more doses of the vaccine suitably formulated may be administered. The vaccine of the present invention may be administered by following conventional schedules.

The treatment may consist in administering one single dose or more subsequent doses. The vaccine according to the present invention may comprise one or more different antigenic components, such as, e.g. tetanus toxoid, diphtheria toxoid or other Bordetella antigens.

In order to assure the best formulations to be enclosed into an anti-pertussis vaccine, the double PT mutant, the FHA and the 69 Kd protein may be stabilized with 0.035% formaldehyde, given as weight / volume (w/v). i.e. corresponding to a weight ratio mutant/formaldehyde of 0.3. Formaldehyde used in such a concentration allows said antigens to be stabilized and therefore to be maintained for long periods of time by preventing the enzymatic degradation without however affecting the immunological properties. For concluding, the PT mutant, the FHA and the 69Kd protein obtained by mutant strains of B. pertussis according to the present invention, represent, because of their high immunogenicity and absence of toxicity, antigens of election for developing acellular anti-pertussis mono- or trivalent (DPT) vaccine showing the desired features.

EXAMPLE 1 :

Production, purification and characterisation of PT-9K/129G.

4

The PT-9K/129G double mutant containing the two amino acid substitutions Arg9 -> Lys and Glu129 -> Gly within the S1 sequence is obtained from cultures of the transformed B. pertussis strain W28-9K/129G. The construction of the transformed bacterium as the production, the purification, and the physico-chemical and immunological properties of the PT-9K/129G double mutant have been disclosed in details in the Italian patent application No. 19286 of 07.02.1990.

As further option to the method disclosed in the aforementioned application, the first purification process may be carried out as an alternative to the adsorption upon Affigel Bleu, by passing the culture supernatant of B. pertussis W28-9K/129G strain, containing the double mutant, through hydroxyapatite according to a modification of the method described by Sato et al. (Infect. Immun. 1983, 41: 313-320).

At the end of the growth by centrifugation of the B.pertussis W28-9K/129G culture, as disclosed in the above cited patent application, the supernatant is filtered in sterile conditions on DuraporeR cartridge (Millipore) 0.22 mμ and then brought to pH 6.65 with 2M HCl. This supernatant, which derives from 20-40 litres culture, is so suitably diluted to have a conductance equivalent to that of a 100 mM NaCl solution, then it is loaded onto a chromatography column containing 500 ml hydroxyapatite (IBF France) (this volume can bound 300-400 mg protein) equilibrated by repeated washes with 10 mM phosphate buffer pH 6.65. After subsequent washes with 70 mM phosphate buffer, pH 6.65, the PT-9K/129G double mutant is eluted at 8°C with 0.25 M phosphate buffer, pH 6.65. The fractions containing the double mutant are collected, put together and submitted to a second purification process by adsorption upon phetuin as disclosed in the IT patent application No. 19286 of 07.02.1990.

EXAMPLE 2:

Characterisation of the PT-9K/129G mutant activities.

The chimical-physical properties and the biological activities of the PT-9K/129G double mutant have been analysed in details in the IT patent application No. 19286 of 07. 02. 1990. A summary of said activities is reported in Table II.

TABLE II

in vivo and in vitro activity of the PT-9K/129G double mutant    and of the PT wild type

---

| Activity | PT | PT-9k/129G |
|---|---|---|
| ADP ribosylation (μg) | 0.001 | > 20 |
| Mitogenicity (μg/ml) | 0-1-0.3 | 0.1-0.3 |
| Haemagglutination (μg/well) | 0.1 | 0.1 |
| Affinity (anti-S1) [Ka(L/mol)] | $2.4 \times 10^8$ | $6.1 \times 10^8$ |
| Affinity (anti-PT) [Ka(L/mol)] | $2 \times 10^{10}$ | $9.8 \times 10^9$ |
| Histamine (sensitisation) (μg/rat) | 0.1-0.5 | > 50 |
| Leucocytose (stimulation)(μg/rat) | 0.02 | > 50 |
| Anaphylaxis (enhancement)(μg/rat) | 0.04 | >7.5 |
| Hyperinsulinemia (induction)(μg/rat) | < 1 | > 25 |
| Toxicity on CHO cells (μg/ml) | 0.005 | > 5000 |
| Acute toxicity in vivo (μg/Kg) | N.D. | > 1500 |

---

N.D.= not determined.

The analysis of PT-9K/129G mutant immunogenicity in guinea-pigs, and protective ability against intracerebral infections from virulent B. pertussis in rats and the results of the clinical experimentation effected on voluntary adults, have been described respectively in example 9, 10 and 11 of the IT patent application 19286 of 07.02.90. A summary of the results is reported in Table III and IV.

TABLE III

Immunogenicity of the PT-9K/129G double mutant

| Protection in rat | | Antibody response in guinea-pigs | | | | |
|---|---|---|---|---|---|---|
| | | ELISA[a] titre | | | Neutralisation[b] titre | |
| Dose (µg) | S/T(c) (µg) | dose (µg) | Post 1/ Pre | Post 2/ Pre | Post 1 | Post 2 |
| 30 | 16/16 | 3 | 35 | 48 | 1/20 | 1/1280 |
| 12 | 16/16 | 10 | 38 | 52 | 1/20 | 1/1280 |
| 4.8 | 12/16 | 25 | 52 | 55 | 1/20 | 1/1280 |
| 1.9 | 10/16 | 50 | 55 | 63 | 1/80 | 1/1280 |
| 0.7 | 7/16 | | | | | |
| 0.2 | 3/16 | | | | | |

a) The titre is expressed as ratio of the ABS-MAXs

b) The titre is expressed as that dilution of guinea-pig serum capable of neutralizing the toxic effect of the native PT on CHO cells.

c) S/T = Survived from a total of 16 rats intracerebrally infected with virulent B. pertussis.

TABLE IV – Antibody and neutralizing titre in sera of voluntary adults receiving placebo or PT-9K/129G vaccine

| Time | Experiment groups | | | |
|---|---|---|---|---|
| | Placebo | | Vaccine | |
| | Geometric mean (95% confidence interval) | Post/pre | Geometric mean (95% confidence interval) | Post/pre |
| **Neutralizing titre (inverse serum dilution in CHO assay)** | | | | |
| Day 0 | 12.8 (7.5–21.6) | | 13.6 (8.6–21.5) | |
| Day +30 | 12.8 (6.7–24.9) | 1.0 | 1,810.2** (737.0–4,446.0) | 133 |
| Day +72 | 11.7 (5.9–23.0) | 0.9 | 1,974.0** (820.1–4,751.4) | 145 |
| **Anti-PT antibody titre (ELISA units per ml)** | | | | |
| Day 0 | 6.5 (3.5–12.2) | | 6.7 (4.6–9.8) | |
| Day +30 | 8.1 (4.2–15.5) | 1.2 | 496.5** (199.9–1,233.3) | 74 |
| Day +72 | 7.9 (4.5–13.9) | 1.2 | 522.4** (216.8–1,258.6) | 78 |
| **Anti-FHA antibody titre (ELISA units per ml)** | | | | |
| Day 0 | 9.4 (4.3–20.7) | | 12.7 (7.5–21.6) | |
| Day +30 | 9.9 (4.7–20.9) | 1.0 | 85.1** (54.9–131.7) | 6.7 |
| Day +72 | 8.4 (4.1–17.3) | 0.9 | 57.7* (39.1–85.5) | 4.5 |

\* $P < 0.05$ vs value day 0.

\*\* $P < 0.01$ vs value day 0

Two further types of experiments not described in the aforementioned patent (application) are hereinafter reported to strengthen the capability of the PT-9K/129G double mutant of eliciting protective antibodies.

A) In vivo neutralisation of leucocytose and sensitisation to histamine.

Groups of female BALB/c mice of about 16-18 gr are intraperitoneally (i.p.) inoculated with 0.5 ml saline solution comprising 3 μg PT-9K/129G adsorbed upon Al(OH)₃ (1 mg/ml). The control mice are i.p. inoculated with physiological solution comprising 0.02-0.1 or 0.5 μg PT. Specimens of blood are collected on day +33 from the orbital plexus of each mouse and the leucocytose is then monitored with a culture counter (Coulter Electronics Ltd, Luton England). The same mice are then inoculated on day + 36 with 4 mg Histamine dihydrochloride (Sigma Chemical Co., St. Louis MO) in 0.5 saline solution and 24 hours later mortality is recorded.

TABLE V

In vivo neutralisation of leucocytose and sensitisation to histamine

| Immunisation | Dose PT (μg/mouse) | leucocytose (PBMC/ml ±SDx10$^6$) | Histamine No. dead/No. total assayed mice |
|---|---|---|---|
| Al(OH)3 | - | 7.7 ± 0.40 | 0/8 |
| | 0.020 | 7.9 ± 0.31 | 2/8 |
| | 0.100 | 16.3 ± 1.32 | 8/8 |
| | 0.500 | 18.6 ± 0.85 | 8/8 |
| Al(OH)3 + | 0.020 | 8.2 ± 0.61 | 0/8 |
| PT-9K/129G | 0.100 | 7.8 ± 0.42 | 0/8 |
| (3 mg/mouse) | 0.500 | 7.5 ± 0.25 | 0/8 |

The results reported in Table V show that 0.1 μg PT are able to induce a dramatic increase in leucocyte rate in peripheral blood and also 100% mortality after histamine challenge in mice injected with Al(OH)₃ only and tested individually. On the contrary neither leucocytose nor sensitisation to histamine was observed in mice immunized with 3 μg PT-9K/129G vaccine.

B) In vitro neutralisation of the enzyme activity of pertussis toxin.

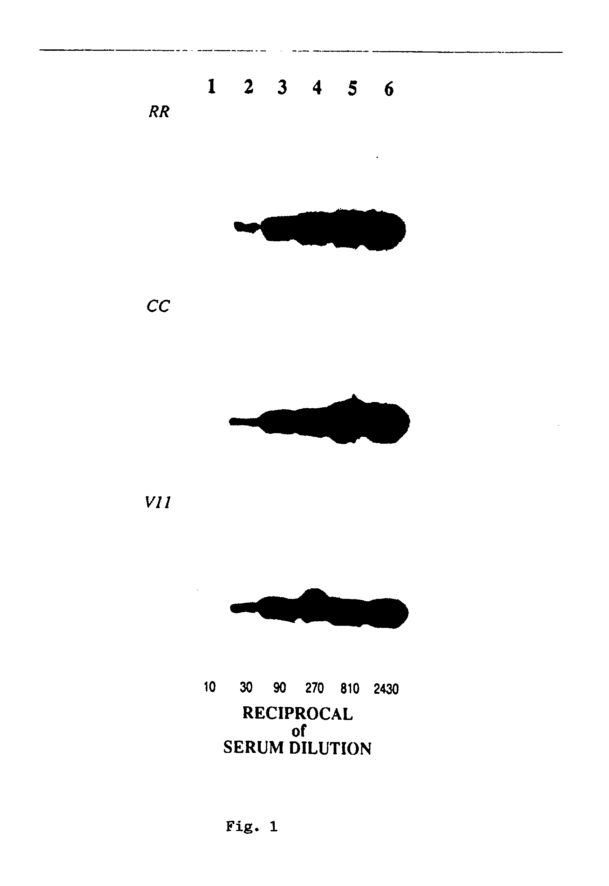

Antibodies from three voluntary adults vaccinated with a dose of PT-9K/129G vaccine and included in the clinical test disclosed in example 11 of the Italian patent application 19286 are assayed for their ability to in vitro neutralize the ADP-ribosyltransferase activity of PT. The test is carried out as previously described (Pizza et al., Proc. Natl. Sci. Acad. USA, 1988, 85:7521-7525) by using the bovine transducine as the substrate of the enzymatic reaction. For performance of the test, 10 μl human serum diluted 1/10, 1/30, 1/90, 1/270, 1/810 and 1/2430 are incubated at 37°C for 60 min. with 5 μl containing 50 ng PT. The mixtures are thereafter preactivated upon incubation for 30 min with 25 mM dithiothreitol at room temperature. At the end of the preactivation period, 5 μl 2M Tris, pH 7.5, 1 μl 100 mM adenosine triphosphate (ATP) solution, 10 μl 25 mM thymidine solution and 10 μl of a membrane mixture of the outer segment of bovine retina comprising transducin are added to the final volume of 100 μl. The reaction mixtures are then incubated for 2 hours at room temperature. Thereafter the mixtures are centrifuged and the pellet comprising the transducin is solubilized in 30 μl sodium dodecylsulphate, loaded onto and run on 12.5% acrylamide gel according to the classical method (Laemmli et al., Nature, 1970, 227:680-685). The radiolabeled transducin is detected by autoradiography. The inhibition of the ADP ribosylation of bovine transducin with [32$_p$] NAD by immunosera from three voluntary adults (RR CC V11) vaccinated with PT-9K/129G is illustrated in Fig.1. The reverse of the serum dilution are reported in lines 1 to 6. For purpose of quantitative analysis, the bands comprising transducin are excised and the radioactivity is counted with a a counter (Beckman). The results given in counts per minute (cpm) are reported in Table VI.

TABLE VI

ln vitro inhibition of the ADP-ribosylating activity of PT by sera

of adult volunteers vaccinated with PT-9K/129G.

| Sera specimen | PT (50 ng) | Serum dilution -1 | | | | | | |
|---|---|---|---|---|---|---|---|---|
| | | $10^a$ | 30 | 90 | 270 | 810 | 2430 | ED$_{50}$ |
| - | 1.264(b) | - | - | - | - | - | - | - |
| RR | | 44 | 62 | 191 | 391 | 467 | 532 | 1,264 |
| CC | | 53 | 82 | 313 | 410 | 978 | 788 | 665 |
| V11 | | 63 | 115 | 461 | 540 | 674 | 799 | 675 |

(a) Reverse of the serum dilution capable of inhibiting the 50% of

the ADP-ribosylation of the [32$_p$] labeled bovine transducin induced

by 50 ng PT.

(b) counts per minute (cpm).

As showed in Figure 1 and in Table VI, the sera of adult volunteers vaccinated with the PT-9K/129G double mutant are capable of completely inhibiting the enzyme activity of 50 ng PT.

EXAMPLE 3:

Production and purification of the filamentous haemagglutinin (FHA).

The FHA is secreted in the culture supernatant of B.pertussis W28-9K/129G which produces the PT-9K/129G double mutant or of B. pertussis W28ΔTox, which does not produce PT and which are both disclosed in the Italian patent application 19286. The protein is purified from the culture supernatant from which also the PT-9K/129G double mutant is purified by using the same column of hydroxyapatite or from the supernatant of the B. pertussis W28ΔTox culture upon ex novo preparation of a hydroxyapatite column. The FHA is eluted with 0.1 M phosphate buffer, pH 8.0 comprising NaCl in end concentration 0.5 M , directly after the elution of the PT-9K/129G double mutant, which is achieved at 8°C with phosphate buffer at ionic strength and pH as reported in example 1 of the present application, . Should on the contrary the FHA be prepared and purified from the supernatant of B. pertussis W28-ΔTox, the supernatant is directly loaded onto the column of hydroxyapatite omitting the purification stage for the PT-9K/129G double mutant and operating as above described. The elution fractions containing the FHA are put together and precipitated at 4°C for one night with ammonium sulphate at the end concentration 70% of saturation. The precipitate is centrifuged and the obtained pellet are suspended again in 50 mM Tris HCl buffer pH 8.0, containing 0.5 M NaCl.

EXAMPLE 4:

Production and purification of the 69 Kd protein.

The 69 Kd protein is obtained by purifying a cellular extract of B. pertussis W28-9K/129G or B. pertussis W28-ΔTox. The cellular extract is prepared essentially according to Brennan et al. [Infect. Immun. 56:3189-3195 (1988)]. The cellular pellet derived from 20-40 litres culture, is suspended in 9 litres 10 mM Tris-HCl buffer, pH 8.0 containing 0.15 M NaCl by heating to 60°C for 1 hour with gentle stirring. After cooling to 10°C, the suspension is centrifuged at 18,000 rpm (rotor JCF-Z, Beckman) and ammonium sulphate is added to the resulting supernatant up to the final concentration 2 M. A further centrifugation at 9,500 rpm is carried out and the pellet is extracted upon suspension in 100 ml of 50 mM diethanolamine solution, pH 8.8 (buffer A) with gentle stirring for 15' at 4°C. The cycle is repeated once again. The centrifuged extracts are dialysed versus buffer A. After centrifugation, the dialysed solution is loaded onto a mono Q preparative column (anion exchange chromatography column from Pharmacia, Uppsala, Sweden) equilibrated with buffer A. The column is eluted with 0.1 M NaCl gradient in buffer A. The 69 Kd protein (OMP) comprising fractions are collected, cooled at 4°C, precipitated by adding ammonium sulphate upto the end concentration 2 M and sedimented for one night. The precipitate is dialysed versus buffer A comprising 300 mM NaCl and gel filtrated on Sephacril HR 200 column (Pharmacia) equilibrated with the same buffer. By following this purification method about 100-120 mg 69Kd protein yield from 20 litres supernatant of both B. pertussis W28-9K/129G and B. pertussis W28-ΔTox culture.

EXAMPLE 5:

Characterisation of the chimico-physical properties of FHA and 69Kd.

A) Electrophoresis profile..

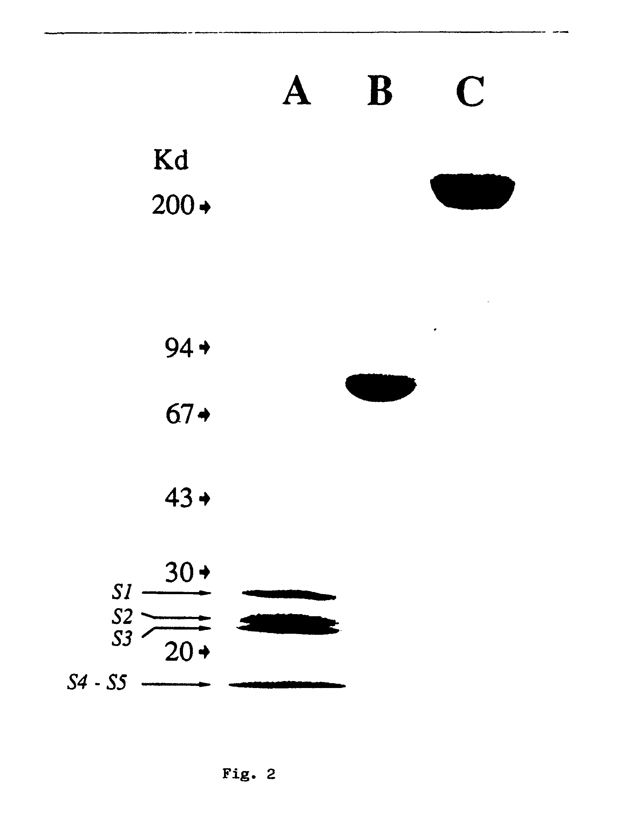

The chimico - physical features of the purified FHA and 69Kd have been determined on sodium dodecyl sulphate (SDS) polyacrylamide gel electrophoresis (8% for the FHA and 12.5% for the 69Kd) as disclosed by Laemmli U.K. (1970) Nature, 227 : 680-685. The gel is stained with Comassie blue R250. The results illustrated in Fig. 2 show the electrophoretic profile of purified PT-9K/129G (line A), 69Kd (line B) and FHA (line C). Bottom left the bands peculiar for the 5 subunits of the PT are reported. As evident, a single band of molecular weight of about 200 Kilodaltons is present in the preparation of FHA (line C) and a single band of molecular weight of about 69 kilodaltons in that of 69Kd (line B). By a more accurate control effected by Wester Blotting analysis (Towbin H.T. et al. Proc. Natl. Acad. Sci. USA, 1976, 73:361-365) using specific anti-PT, anti-FHA and anti-69Kd antibodies, any contaminating proteins have been proved to be completely absent from the FHA and 69Kd preparation. The FHA and 69Kd proteins exhibit a purity of 99%.

B) Analysis of the amino acid composition.

The analysis of the amino acid residues of FHA and 69Kd is made as described by Spackman D.H. et al. Anal. Chem. 1958, 30: 1190 - 1206. The acid hydrolysis of the proteins is carried out in 6N HCl, at 110°C for 24 hours in vials sealed in vacuum. Thereafter the analysis is effected in amino acid analyser (Kontron, Zurig, Swizerland). The values reported in Table VII and given as amino acid / protein molar ratio are in agreement with the theoretic values derived from the primary structure of the above cited proteins.

TABLE VII.

Amino acid composition of the purified FHA and 69Kd.

| Amino acid | Molar ratio amino acid/protein | | | |
| | Theoretic [a] | | Found [b] | |
| | FHA | 69Kd | FHA | 69Kd |
|---|---|---|---|---|
| Asp | 227 | 59 | 227.7 | 57.6 |
| Thr | 128 | 38 | 122.5 | 38.3 |
| Ser | 168 | 43 | 146.7 | 42.2 |
| Glx | 185 | 57 | 188.3 | 60.9 |
| Pro | 45 | 43 | 54.71 | 52.8 |
| Gly | 296 | 101 | 292.2 | 88.9 |
| Ala | 339 | 97 | 331.0 | 91.7 |
| $1/2(Cys)_2$ | 3 | 0 | - | - |
| Val | 208 | 56 | 206 | 58.7 |
| Met | 25 | 4 | 28.7 | 3.9 |
| Ile | 83 | 22 | 76.4 | 23.3 |
| Leu | 198 | 61 | 199.8 | 61.1 |
| Tyr | 29 | 7 | 29.2 | 6.9 |
| Phe | 27 | 14 | 26.3 | 12.3 |
| NH3 | 200 | 51 | 191.4 | 52.4 |
| Lys | 98 | 14 | 97.7 | 13.7 |
| His | 41 | 11 | 36.6 | 11.9 |
| Arg | 126 | 40 | 129.3 | 35.7 |

a) Theoretic values derived from the protein primary structure;

b) Amino acid residues found from the purified preparations.

EXAMPLE 6:

In vitro characterisation of FHA.

A) Haemagglutination:

The haemagglutination assay is carried out as described by Sato Y.et al. Infect. Immun., 1983, 41: 313-320 using glutaraldehyde fixed chicken erythrocytes as target cells. The results given as that FHA amount provoking the complete erythrocytes agglutination show that the minimum agglutinating dose is 100-150 ng/well.

EXAMPLE 7:

Formaldehyde treatment of FHA and 69Kd.

The FHA and 69 Kd purified as respectively described in example 3 and 4 are dialysed versus PBS, pH 7.4 comprising 0.025 M lysine (Ajinomoto, Tokio, Japan) and 0.01% merthiolate (ELANCO, USA), at 4°C for 24 hours and then brought again in PBS solution. After determination of the protein content (Lowry O.H. et al. J. Biol. Chem. , 1951, 193 : 265-275) the mixtures are added with formaldehyde (4% PBS solution containing 0.5 M NaCl, pH 7.4) in such an amount to obtain a final ratio protein/formaldehyde of 0.3. The resulting mixtures are incubated at 37°C for 48 hours and then repeatedly dialysed versus PBS. Thereafter the mixtures are tested to determine the free formaldehyde content, which appears to be lower than 0.01% (weight / volume). The so stabilized FHA exhibits the same haemagglutinating activity of the native molecules. Furthermore a main band of 200 Kd and some higher molecular weight bands derived from the formaldehyde treatment are showed on polyacrylamide gel electrophoresis. Due to the formaldehyde stabilisation, the characteristic FHA degradation is not observed any more. On the other hand the FHA not stabilized with formaldehyde and kept at 4°C or in frozen state, exhibits an electrophoretic profile characterized by a progressive loss of the 200 Kd band and appearance of low molecular weight degradation products (Figure 2, line 3), which have been proved in Western immunoblotting analysis to derive from the FHA main band of 200 Kd. With regard to the non-stabilized protein, the 69Kd protein shows a light, though more expanded band of 69 Kd due to the formaldehyde treatment. The formaldehyde treated 69Kd protein is stable at 37°C for 30 days. On the contrary the non-treated protein maintained at 4°C and 20°C for 15 days is prone to a progressive degradation.

EXAMPLE 8:

Development of acellular anti-pertussis vaccines.

A) Formulation of monovalent vaccines.

Composition dose

| Vaccine lot | PT-9K/129G (µg) | FHA (µg) | 69Kd (µg) | Al(OH)$_3$ (mg) |
|---|---|---|---|---|
| A (D5/FA) | 15 | - | - | 0.5 |
| B (PT-FHA/1) | 10 | 15 | - | 0.5 |
| C (PFK/1) | 7.5 | 10 | 10 | 2 |
| D (PFK/2) | 7.5 | 10 | 10 | 0.5 |
| E (D9/Fa) | 5 | - | - | 0.5 |
| G (D9/Fb) | 10 | - | - | 0.5 |
| H (PFK/4) | 7.5 | 5 | 5 | 0.5 |

B) Formulation of trivalent vaccines

Composition dose

| Vaccine Lot | PT-9K/129G (µg) | FHA (µg) | 69KD (µg) | Diphth. toxoid (Lf) | Tetan. toxoid (Lf) | Al(OH)$_3$ (mg) | AlPO$_4$ (mgAl) |
|---|---|---|---|---|---|---|---|
| A(DPT1/P/A) | 15 | - | - | 30 | 15 | 2 | - |
| B(DPT/H) | 5 | - | - | 15 | 10 | 2 | - |
| C(DPT/M) | 10 | - | - | 15 | 10 | 2 | - |
| D(DPT/A4) | 15 | - | - | 15 | 10 | 2 | - |
| E(DPT2/PF/A) | 10 | 15 | - | 30 | 15 | 2 | - |
| F(DPT/AF/1) | 15 | - | - | 15 | 10 | 1 | 0.3 |
| G(DPT4/P/AH) | 10 | - | - | 15 | 10 | 1 | - |
| H(DPT7/PFK/AH) | 5 | 5 | 3 | 20 | 12.5 | 1 | - |
| I(DPT8/PFK/AF) | 5 | 5 | 5 | 15 | 10 | - | 0.3 |
| L(DPT5/P/AF) | 10 | - | - | 15 | 10 | - | 0.3 |
| M(DPT/G)(a) | 10 | - | - | 15 | 10 | 1 | - |
| N(DPT6/P/AH) | 10 | - | - | 20 | 12.5 | 1 | - |
| O(DPT9/PFK/AH) | 5 | 5 | 3 | 20 | 12.5 | 2 | - |
| P(DPT10/PFK/AH) | 5 | 2.5 | 2.5 | 20 | 12.5 | 1 | - |
| Q(DPT11/PFK/AH) | 5 | 2.5 | 2.5 | 20 | 12.5 | - | - |

a) PT-9K/129G treated with 0.07% formaldehyde corresponding to a protein/formaldehyde ratio of 0.15.

EXAMPLE 9:

In vivo preclinical experimentation of acellular antipertussis vaccines.

A) Immunogenicity analysis in guinea pig.

In order to investigate the immunogenicity of acellular vaccines, groups of 6 four week old guinea pigs, of 350 g, are subcutaneously (s.c.) inoculated with 0.5 ml sterile pyrogen-free saline solution comprising 0.001 mg sodium-ethyl-mercury-thiosalicylate and different doses of adsorbed vaccine. Four weeks after the prime shot, blood specimens are drawn and the animals are s.c. boosted with the same dose of vaccine. Two weeks after the first booster blood specimens are drawn again. The sera obtained from the drawn specimens are then tested by ELISA assay to determine the anti-PT, anti-FHA and anti-69Kd antibody titre, and by CHO assay to check the anti-body ability to neutralize the wild type PT toxicity. The ELISA assay, which is a modified form of the assay described by Engvall and Perlmann (J. Immunol., 109:129-135, 1972) is accomplished as follows. Each flat bottomed well of a poly-styrene microdish (Dynatech, Laboratories, Inc., Alexandria, VA) is given 200 $\mu$l PBS, pH 7.4, comprising 1 $\mu$g PT or 1 $\mu$g FHA or 1 $\mu$g purified 69Kd. The antigen adsorption upon the solid phase is carried out in a humidified chamber at 37°C for 3 hours and following 4°C for a night. After addition of 1% BSA in PBS to inhibit the nonspecific binding of the seric proteins to the solid support, the guineas pig serum tests serially diluted with PBS added with 0.05% Tween-20 up to 1:9120 are fed into each well of the microdishes and incubated at 37°C for 2 hours. Finally, alkaline phosphatase (Miles, Yeda, Israel) labeled goat anti-guinea pig IgG antibodies, diluted 1:3000 with PBS comprising 0.05% Tween-20, are added to each well and the dishes incubated at 37°C for 2 hours. 100 $\mu$l volumes are used in all the steps and 200 $\mu$l for washing the dishes between two subsequent incubation. Washes are carried out in triplicate using PBS comprising 0.05% tween-20 and 0.02% NaN$_3$. The colorimetric reaction developing at room temperature in 30 minutes after addition of the specific substrate (p-nitrophenylphosphate, 1 mg/ml) is monitored at 405 nm with Titertec Multiskan (Flow Laboratories, McLean, VA). Control blanks in each dish include wells containing serum tests free of the antigen and vice versa.

The antibody titres are evaluated by recording on flow chart the dilutions of the serum tested in duplicate (abscissa) versus the mean of the corresponding absorbance (ordinate). The intersection between the line through the flex point and the absorbance axis gives the value of the undiluted serum absorbance (ABS-MAX). The ABS-MAX obtained for each group of animals immunized with varying antigen amounts are compared after each drawing to the corresponding pre-immunosera. The ABS-MAX increase is regarded as statistically significant when at least four time higher than the value of pre-vaccination serum. The results obtained from some acellular vaccine lots are reported in table VIII.

TABLE VIII.

Antibody titre (IgG ELISA) and neutralisation titre (CHO) of sera from guinea pigs immunized with one or two doses of trivalent acellular DPT1/P/A and DPT2/PF/A vaccines

| | | ELISA[a] titre | | | | Neutralisation[b] titre | |
| | | Post 1/Pre | | Post 2/pre | | Post 1 | Post 2 |
| Vaccine | Dose(µg) PT/FHA | PT | FHA | PT | FHA | PT | PT |
| DPT1/P/A | 15/- | 24 | - | 29 | - | 1/80 | 1/640 |
| | 5/- | 23 | - | 28 | - | 1/20 | 1/320 |
| | 1.6/- | 21 | - | 23 | - | 1/10 | 1/160 |
| DPT2/PF/A | 10/15 | 29 | 36 | 34 | 44 | 1/20 | 1/320 |
| | 3.3/5 | 25 | 34 | 30 | 40 | 1/20 | 1/160 |
| | 1.1/1.6 | 23 | 33 | 26 | 38 | 1/10 | 1/80 |

a) The ELISA titre is displayed as ABS-MAX of the undiluted serum.

b) The neutralisation titre is displayed as that guinea pig serum dilution capable of neutralizing the PT wild type toxic effect on CHO cells.

As it may be appreciated from Table VIII, the sera drawn four weeks after the primer exhibit absorbance values (ABS-MAX) at 405 nm, which are, depending on the applied vaccine and on the injected dose, about 21-24 and 23-29 times (anti-PT) and 33-36 times (anti-FHA) higher than those monitored before immunisation. A further increase is observed for the sera drawn two weeks after the booster. The antibody titre increase observed after immunisation with acellular vaccines correlates to the neutralizing activity as tested with the CHO assay carried out according to Gillenius P. et al. J. Biol. Stand., 1985, 13 : 61-66; Gandstrom M. et al. J. Infect. Dis., 1985, 151 : 646-649. As a matter of fact the sera elicited after one single injection are capable of neutralizing the CHO cell agglutinating effect due to 120 pg PT. The toxin neutralizing ability dramatically increase after booster.

B) Analysis of the effectiveness of acellular anti-pertussis vaccines in mice.

Groups of 16 three-four weeks old male CD1 mice (Charles River, Calco Italy), weighing (about) 16 g, are i.p. injected, according to the WHO regulation, with 0.5 ml sterile saline solution comprising different amounts of PT-9K/129G, FHA and 69Kd depending on the formulation of the vaccine adsorbed upon adjuvant. As positive control mouse groups are i.p. injected with 0.00032, 0.001, 0.008 and 0.04 ml standard cellular anti-pertussis vaccine (National Institute of Health, Bethesda, MD). 14 day after the injection the mice are intracerebrally (IC) challenged with a virulent B. pertussis suspension (strain 18323, Sclavo S.p.A.) comprising 300 LD50. Then mice have been kept under control 14 days. The anti-tetanus effectiveness is evaluated in mouse while the anti-diphtheria effectiveness is evaluated in guinea pigs according to the WHO regulation. The results of the anti-tetanus and anti-diphtheria activity are displayed as international units per ml (UI/ml) with reference to the standard cellular vaccine. The results from some lots referred to in example 8 are reported in Table IX and X.

```
        TABLE IX.

        Anti-pertussis activity of acellular monovalent  adsorbed  vaccines

        in mouse (Item A, example 8).
```

| Cellular vaccine [a] | | lot | Acellular vaccine | | | Surviv. [b] |
|---|---|---|---|---|---|---|
| Dose (ml) | surviv. | | PT-9K/129G (µg) | FHA (µg) | 69Kd (µg) | |
| 0.04 | 16/16 | A | 30 | - | - | 14/16 |
| 0.008 | 13/16 | | 6 | - | - | 14/16 |
| 0.001 | 9/16 | | 1.2 | - | - | 8/16 |
| 0.0003 | 1/16 | | 0.24 | - | - | 2/16 |
| 0.04 | 12/16 | B | 10 | 15 | - | 7/16 |
| 0.008 | 6/16 | | 3.3 | 5 | - | 6/16 |
| 0.001 | 3/16 | | 1.1 | 1.6 | - | 7/16 |
| 0.0003 | 0/16 | | 0.3 | 0.5 | - | 2/16 |
| 0.04 | 10/16 | C | 7.5 | 10 | 10 | 15/16 |
| 0.008 | 2/16 | | 2.5 | 3.3 | 3.3 | 15/16 |
| 0.001 | 0/16 | | 0.83 | 1.1 | 1.1 | 12/16 |
| 0.04 | 10/16 | D | 7.5 | 10 | 10 | 15/16 |
| 0.008 | 2/16 | | 2.5 | 3.3 | 3.3 | 15/16 |
| 0.001 | 0/16 | | 0.83 | 1.1 | 1.1 | 12/16 |
| 0.04 | 9/16 | E | 5 | - | - | 11/16 |
| 0.008 | 4/16 | | 1 | - | - | 1/16 |
| 0.001 | 0/16 | | 0.2 | - | - | 0/16 |
| 0.04 | 9/16 | F | 10 | - | - | 10/16 |
| 0.008 | 4/16 | | 2 | - | - | 1/16 |
| 0.001 | 0/16 | | 0.4 | - | - | 0/16 |

Lot H: for the time being, under test.

a) Vaccine comprising 8 protective international units per ml (UI/ml); b) Values displayed as number of survived mice out of a total of 16 mice intracerebrally infected.

TABLE X.

Anti-pertussis activity of acellular trivalent (DPT) adsorbed vaccines in mouse.

| Cellular vaccine[a] | | | Acellular vaccine | | | | | |
| dose (ml) | surv. | lot | PT-9K/ 129G (µg) | FHA (µg) | 69Kd (µg) | Diph. Tox. (Lf) | Tet. Tox. (Lf) | surv.[b] |
| --- | --- | --- | --- | --- | --- | --- | --- | --- |
| 0.04 | 9/16 | A | 15 | - | - | 30 | 15 | 6/16 |
| 0.008 | 2/16 | | 5 | - | - | 10 | 5 | 5/16 |
| 0.001 | 0/16 | | 1.66 | - | - | 3.3 | 1.66 | 4/16 |
| | | | | | | --- | --- | |
| | | | | | | 81[c] | 762[d] | |
| 0.04 | 9/16 | B | 5 | - | - | 15 | 10 | 9/16 |
| 0.008 | 4/16 | | 1 | - | - | 3 | 2 | 4/16 |
| 0.001 | 0/16 | | 0.2 | - | - | 0.6 | 0.4 | 0/16 |
| | | | | | | --- | --- | |
| | | | | | | 100[c] | 237[d] | |
| 0.04 | 9/16 | C | 10 | - | - | 15 | 10 | 10/16 |
| 0.008 | 4/16 | | 2 | - | - | 3 | 2 | 5/16 |
| 0.001 | 0/16 | | 0.4 | - | - | 0.6 | 0.4 | 1/16 |
| | | | | | | --- | --- | |
| | | | | | | 96[c] | 216[d] | |
| 0.04 | 14/16 | D | 15 | - | - | 15 | 10 | 6/16 |
| 0.008 | 9/16 | | 5 | - | - | 5 | 33 | 10/16 |
| 0.001 | 0/16 | | 1.6 | - | - | 1.6 | 1.1 | 8/16 |
| | | | | | | --- | --- | |
| | | | | | | 89[c] | 485[d] | |

| | | | | | | | | |
|---|---|---|---|---|---|---|---|---|
| 0.04 | 9/16 | E | 10 | 15 | - | 30 | 15 | 8/16 |
| 0.008 | 2/16 | | 3.3 | 5 | - | 10 | 5 | 11/16 |
| 0.001 | 0/16 | | 1.1 | 1.6 | - | 3.3 | 1.66 | 10/16 |
| | | | | | | --- | --- | |
| | | | | | | 76[c] | 1115[d] | |
| 0.04 | 14/16 | F | 15 | - | - | 15 | 10 | 6/16 |
| 0.008 | 9/16 | | 5 | - | - | 5 | 3.3 | 8/16 |
| 0.001 | 0/16 | | 1.6 | - | - | 1.6 | 1.1 | 8/16 |
| | | | | | | --- | --- | |
| | | | | | | 93[c] | 273[d] | |
| 0.04 | 16/16 | M | 15 | - | - | 15 | 10 | 15/16 |
| 0.008 | 7/16 | | 5 | - | - | 5 | 3.3 | 15/16 |
| 0.001 | 0/16 | | 1.6 | - | - | 1.6 | 1.1 | 7/16 |
| | | | 0.5 | - | - | 0.5 | 0.3 | 1/16 |
| | | | | | | --- | --- | |
| | | | | | | ND[e] | 281[d] | |

----------------------------------------------------------------

Lots G, H, I, L, N, O, P, Q, for the time being under test.

a)   vaccine   comprising 8 UI/ml;

b) values displayed as number of survived mice out of a total of 16 tested mice;

c) anti-diphtheria titre given in  UI/ml;

d) anti-tetanus titre given in UI/ml;

e) ND = Not determined (under test).

The bulk preparation of the different lots of adsorbed vaccine is achieved in sterile vessels of pyrex glass fitted with stirrer, by mixing the suitable amounts of the different antigenic compounds, of aluminium or phosphate hydroxide as an adjuvant and of sterile pyrogen-free saline solution comprising 0.01% sodium-ethyl-mercury-thiosalicylate as pre-servative, in order to obtain the desired formulation per dose. After thorough mixing an assay of the suspension is collected for the sterility control. Upon favourable result, each vaccine lot is forwarded to the dispensing department. The filling in the final vessels (0.5 ml/phial) is performed in a suitable department including a sterile unit and equipped with automatically programmed sterilizers, temperature recorders and cooling means. The filling and the sealing of the final phials is accomplished with a closed-phial-dispenser and an automatic welder device. The product in phials is forwarded to the cooled unit for the quarantine (refrigerator at +5°C) waiting for the results of sterility, quality and quantity controls.

## Claims

1. Acellular anti-pertussis vaccines comprising a non-toxic double mutant of pertussis toxin, filamentous haemagglutinin (FHA), 69 kilodaltons protein (69Kd) and pharmaceutically acceptable vectors optionally in association with diphtheria and/or tetanus anatoxin.

2. Acellular anti-pertussis vaccines according to claim 1, wherein the filamentous haemagglutinin (FHA) and the 69 Kilodaltons protein (69Kd) are obtained from a Bordetella pertussis strain in which the pertussis toxin gene is deleted (B. pertussis ΔTox) or so modified to produce a non-toxic PT.

3. Vaccines according to claims 1 and 2 wherein the non-toxic mutant of the pertussis toxin comprises in the amino acid sequence of the S1 subunit the substitution of Glu 129 and at least one of any other amino acid of the subunit, whose substitution brings about a decrease in the mutant toxicity, by natural amino acids.

4. Vaccines according to claim 3, wherein Glu 129 in the amino acid sequence of the S1 subunit is replaced by Gly.

5. Vaccines according to claim 4, wherein the amino acid Arg 9 in the amino acid sequence of the S1 subunit is replaced

6. Vaccines according to claim 5, wherein Arg 9 is replaced by Lys.

7. Vaccines according to claim 4, wherein the amino acid Trp 26 in the amino acid sequence of the S1 subunit is replaced.

8. Vaccines according to claim 7, wherein Trp 26 is replaced by Ile.

9. Vaccines according to claim 4, wherein the amino acid Arg 13 in the amino acid sequence of the S1 subunit is replaced.

10. Vaccines according to claim 9, wherein Arg 13 is replaced by Leu.

11. Vaccines according to claim 4, wherein the amino acids Asp 11 and Trp 26 in the amino acid sequence of the S1 subunit are replaced.

12. Vaccines according to claim 11, wherein Asp 11 is replaced by Ser and Trp 26 by Ile.

13. Non-toxic mutant of the pertussis toxin comprising in the amino acid sequence of the S1 subunit the substitution of Glu 129 and at least one of any other amino acid of the subunit, whose substitution brings about a decrease in the mutant toxicity, by natural amino acids.

14. Mutant according to claim 13, wherein Glu 129 is replaced by Gly.

15. Mutants according to claims 13 and 14 stabilized by treatment with 0.035% to 0.7% formaldehyde (percent weight/volume).

16. Bordetella pertussis strains wherein the chromosomal pertussis gene encodes the mutants according to claims 13 and 14.

17. Process for the preparation of FHA and 69Kd wherein a Bordetella pertussis strain is used in which the pertussis toxin gene is deleted (B.pertussis ΔTox) or so modified to produce a non-toxic PT.

18. Process for the preparation of FHA and 69Kd wherein a strain according to claim 16 is grown in a liquid culture medium and the so obtained antigens are isolated and purified.

19. Use of the antigens obtained according to the process of claim 18 as active agents for manufacturing monovalent acellular anti-pertussis vaccines.

Fig. 1

Fig. 2